# EUROPEAN PATENT APPLICATION

(11) **EP 1 396 244 A2**
(43) Date of publication of application: **10.03.2004**
(21) Application number: 03020205.5
(22) Date of filing: 05.09.2003
(51) Int. Cl.: A61F 9/01

(54) **Corneal-ablation-data calculation apparatus and corneal surgery apparatus**

(30) Priority: 06.09.2002 JP 2002261060; 04.12.2002 JP 2002352920
(71) Applicant: Nidek Co., Ltd., Gamagori-shi, Aichi-ken 443-0035 (JP); TELANDRO, ALAIN, 06400 CANNES (FR)
(72) Inventor: Suzuki, Yoshitaka, Okazaki-shi Aichi-ken, 444-2121 (JP); Telandro, Alain, 06400 Cannes (FR)
(74) Representative: Winter, Brandl, Fürniss, Hübner, Röss, Kaiser, Polte Partnerschaft

(57) **Abstract**

A corneal-ablation-data calculation apparatus and a corneal surgery apparatus which enable presbyopic correction with which a post-operative condition of visibility becomes favorable. An apparatus for calculating data on corneal ablation for corneal surgery for correcting a refractive error by ablating corneal tissue with a laser beam includes a first input device for inputting a pre-operative corneal shape, a second input device for inputting a correction amount for far vision and additional power for near vision, a setting device for setting a far vision area in a central part of a cornea and an annular near vision area outside the far vision area, and a calculation device for obtaining the data on the corneal ablation based on the inputted pre-operative corneal shape, the inputted correction amount for far vision and the inputted additional power for near vision, and the set far vision area and the set near vision area.

## Description

### BACKGROUND OF THE INVENTION

### 1. Field of the Invention

The present invention relates to a corneal-ablation-data calculation apparatus and a corneal surgery apparatus for use in corneal surgery for correcting a refractive error by ablating corneal tissue with a laser beam, more specifically relates to an apparatus suitable for presbyopic correction.

### 2. Description of Related Art

As a refractive error of an eye, presbyopia that it becomes harder to see a near point with age is mentioned in addition to myopia, astigmatism and hyperopia. Conventionally, as a method for presbyopic correction, spectacles are generally used for the correction. Recently, an apparatus which ablates a cornea by using a laser beam and changes a corneal curvature to correct the refractive error such as myopia, astigmatism or hyperopia has been introduced. In addition, a study for correcting presbyopia using that corneal surgery apparatus is conducted. For example, there is proposed a method for forming an area for near vision partly in a position deviated from a corneal center so that the cornea may become bifocal.

However, in such method of presbyopic correction, as a jump of an image occurs between an area for far vision and the area for near vision, there is a case where the post-operative condition of visibility is not favorable.

### SUMMARY OF THE INVENTION

An object of the invention is to overcome the problems described above and to provide a corneal-ablation-data calculation apparatus and a corneal surgery apparatus which enable presbyopic correction with which a post-operative condition of visibility becomes favorable.

To achieve the objects and in accordance with the purpose of the present invention, an apparatus for calculating data on corneal ablation for corneal surgery for correcting a refractive error by ablating corneal tissue with a laser beam has a first input device for inputting a pre-operative corneal shape, a second input device for inputting a correction amount for far vision and additional power for near vision, a setting device for setting a far vision area in a central part of a cornea and an annular near vision area outside the far vision area, and a calculation device for obtaining the data on the corneal ablation based on the inputted pre-operative corneal shape, the inputted correction amount for far vision and the inputted additional power for near vision, and the set far vision area and the set near vision area.

In another embodiment of the present invention, a corneal surgery apparatus for correcting a refractive error by ablating corneal tissue with a laser beam has a laser irradiation system having an irradiation optical system for irradiating the laser beam onto a cornea, a first input device for inputting a pre-operative corneal shape, a second input device for inputting a correction amount for far vision and additional power for near vision, a setting device for setting a far vision area in a central part of the cornea and an annular near vision area outside the far vision area, a calculation device for obtaining data on corneal ablation based on the inputted pre-operative corneal shape, the inputted correction amount for far vision and the inputted additional power for near vision, and the set far vision area and the set near vision area, and a control device for controlling the laser irradiation system based on the obtained data on the corneal ablation.

Yet, in another embodiment of the present invention, a corneal surgery apparatus for correcting a refractive error by ablating corneal tissue with a laser beam further has a laser irradiation system having a laser source and an irradiation optical system for irradiating the laser beam onto a cornea, and a mask plate having a light shielding area corresponding to a far vision area in a central part and a first opening corresponding to a near vision area outside the light shielding area, the first opening having a shape where a ratio of a circumferential length at a radius position of the mask plate is made smaller as nearing a center of the mask plate.

Additional objects and advantages of the invention are set forth in the description which follows, are obvious from the description, or may be learned by practicing the invention. The objects and advantages of the invention may be realized and attained by the corneal-ablation-data calculation apparatus and the corneal surgery apparatus in the claims.

### BRIEF DESCRIPTION OF THE DRAWINGS

The accompanying drawings, which are incorporated in and constitute a part of this specification, illustrate embodiments of the present invention and, together with the description, serve to explain the objects, advantages and principles of the invention. In the drawings,
Fig. 1 is a view showing a schematic configuration of a laser irradiation optical system and a control system in a corneal surgery apparatus consistent with the present invention;
Figs. 2A and 2B are views illustrating ablation for presbyopic correction;
Figs. 3A and 3B are views illustrating ablation at each stage in hyperopic presbyopic correction;
Figs. 4A to 4C are views illustrating ablation at each stage in myopic presbyopic correction;
Fig. 5 is a view illustrating a method for forming a transition zone by ablation for myopic correction;
Fig. 6 shows results of ablation provided on a hyperopic presbyopic eye;
Fig. 7 shows results of ablation provided on a myopic presbyopic eye;
Fig. 8 is a view showing a schematic configuration of the corneal surgery apparatus in a case where a mask plate is disposed on a cornea;
Fig. 9A and 9B are views showing a schematic configuration of a mask unit;
Fig. 10 is a view illustrating a shape of the mask plate;
Fig. 11 is a view illustrating a ratio of data on ablation depth distribution in a certain myopic correction amount; and
Fig. 12 is a view illustrating different shapes of the mask plate.

### DETAILED DESCRIPTION OF THE PREFERRED EMBODIMENTS

A detailed description of one preferred embodiment of a corneal-ablation-amount calculation apparatus and a corneal surgery apparatus embodied by the present invention is provided below with reference to the accompanying drawings.

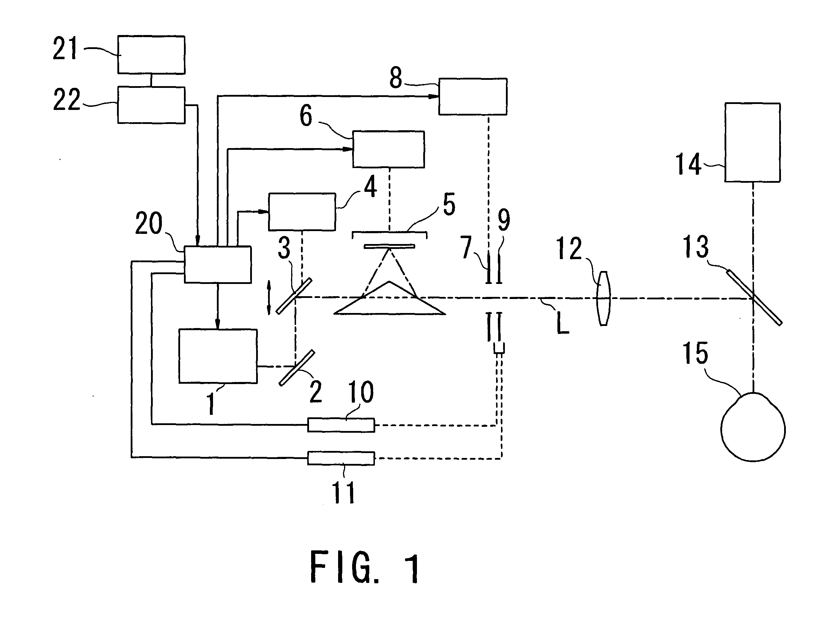

Fig. 1 is a view showing a schematic configuration of a laser irradiation optical system and a control system in a corneal surgery apparatus consistent with the present invention.

A laser beam emitted from a laser source 1 (an excimer laser beam having a wavelength of 193 nm is used in the embodiment) is a pulsed beam, and in its typical shape, intensity distribution of the beam is approximately uniform distribution in a horizontal direction, and is the Gaussian distribution in a vertical direction. In addition, a cross section thereof on a plane intersecting at right angles with respect to a central optical axis L of the irradiation optical system is in a narrow rectangular shape.

The laser beam emitted from the laser source 1 is reflected by a plane mirror 2, and further reflected by a plane mirror 3. The mirror 3 is translatable (movable) in the direction of the arrow shown in Fig. 1 by a driving unit 4, and translates (scans) the laser beam in the Gaussian distribution direction so that an object may be uniformly ablated.

An image rotator 5 is rotated about the optical axis L as its center by a driving unit 6 to rotate the laser beam about the optical axis L. Reference numeral 7 is a circular aperture having a circular opening for limiting an ablation area to a circular shape, and its opening diameter is changed by a driving unit 8. Reference numeral 9 is a slit aperture having a slit opening for limiting an ablation area to a slit shape, and its opening width is changed by a driving unit 10. In addition, the slit aperture 9 is rotated about the optical axis L by a driving unit 11 and its opening direction is changed. A projecting lens 12 projects the openings of the circular aperture 7 and/or the slit aperture 9 onto a cornea 15 of a patient's eye.

Reference numeral 13 is a dichroic mirror having a property of reflecting the excimer laser beam and transmitting visible light. The laser beam passed through the projecting lens 12 is reflected by the dichroic mirror 13, and directed to and irradiated onto the cornea 15.

Placed above the dichroic mirror 13 is an observation optical system 14 having a surgical microscope of a binocular type (commercially available one may be used). Besides, the patient's eye previously goes through positioning in a predetermined position on the occasion of surgery, and the positioning condition is maintained by continuously looking at a fixation target (a fixation light source) not illustrated.

A data input unit 21 inputs data necessary for refractive correction of the patient's eye, and a calculation unit (apparatus) 22 obtains data on corneal ablation (data on an ablation position, an ablation amount and the like) based on the inputted data. A control unit 20 controls the laser source 1, each of the driving units 4, 6, 8, 10 and 11 based on the data on the corneal ablation obtained by the calculation unit 22. Incidentally, a commercially available personal computer may be used for the data input unit 21 and the calculation unit 22.

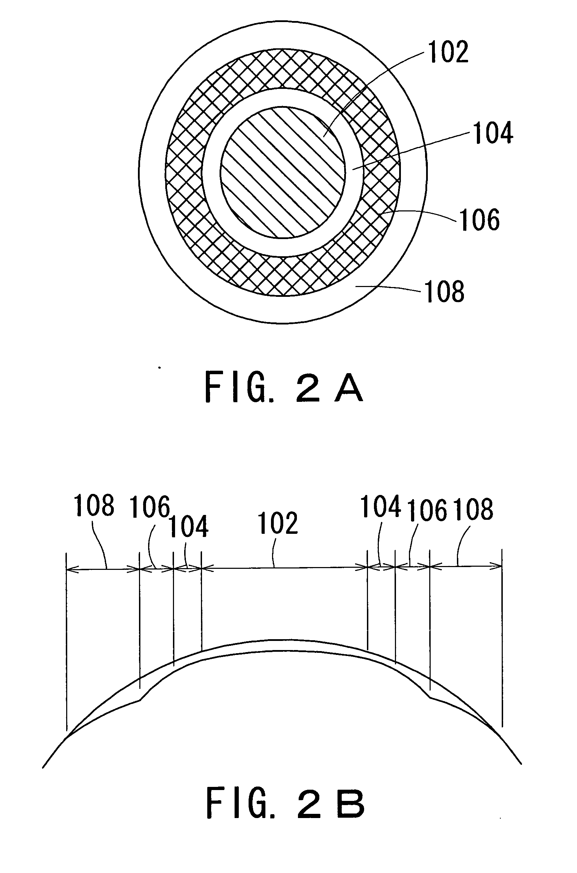

Next, ablation for presbyopic correction will be described. In the presbyopic correction, as shown in Figs. 2A and 2B, an area 102 for far vision (hereinafter referred to as a "far vision area") is set in a central part of the cornea, and an area 106 for near vision (hereinafter referred to as a "near vision area") in an annular shape is set around (outside) the far vision area 102. Also, outside the near vision area 106, a transition zone 108 which smoothly connects the ablation area and a non-ablation area is set. In addition, it is preferable that a blend zone 104 for middle vision is further set between the far vision area 102 and the near vision area 106. The blend zone 104 smoothly connects the far vision area 102 and the near vision area 106. The ablation to form the far vision area 102 and the near vision area 106 is implemented by multi-stage operation. The data necessary for presbyopic correction as above are a pre-operative corneal shape, sizes of the far and near vision areas, a correction amount for far vision, and additional power (diopter) for near vision, and they are inputted by the data input unit 21. The calculation unit 22 obtains a post-operative corneal shape based on the inputted data to calculate the data on the corneal ablation, and the control unit 20 (or the calculation unit 22) obtains data for controlling a laser irradiation system (the laser irradiation optical system and the control system thereof).

In the apparatus in the present embodiment, ablation for myopic correction and ablation for hyperopic correction are combined to form the far vision area 102 and the near vision area 106. Thus, firstly, a brief description will be given on ablations for myopic correction, hyperopic correction and astigmatic correction, respectively, performed by the laser irradiation system in the present apparatus.

### (Ablation for Myopic Correction)

In the case of the myopic correction, the laser beam of which the beam cross section is in the rectangular shape is moved (scanned) within the opening of the circular aperture 7 in the Gaussian distribution direction by the movement of the mirror 3. Then, every time the laser beam has been moved (scanned) in one direction (one scan), movement (scanning) direction of the laser beam is changed by the rotation of the image rotator 5 (for example, in three directions at intervals of 120 degrees, preferably, in different angle direction at each scan) to perform approximately uniform ablation of the cornea within the opening of the circular aperture 7. These processes are performed every time the opening diameter of the circular aperture 7 is sequentially changed. Thereby, the ablation for myopic correction may be performed deeply at the central part of the cornea and shallowly at the peripheral part (for more detail, see Japanese Patent Application Unexamined Publication No. Hei6-114083 corresponding to U.S. Patent No. 5,637,109) .

### (Ablation for Hyperopic Correction)

In the case of the hyperopic correction, the opening diameter of the circular aperture 7 is fixed in alignment with an optical zone (an optical area where the correction is secured). The mirror 3 is moved and fixed so that the laser beam is deviated with respect to the optical axis L, and the laser beam of which the beam cross section is in the rectangular shape is moved (scanned) in an annular shape by the rotation of the image rotator 5 to ablate the cornea in an annular shape. Then, the mirror 3 is sequentially moved to increase the number of irradiation pulses (irradiation time) as the deviation amount of the laser beam from the optical axis L increases. Thereby, the ablation for hyperopic correction may be performed shallowly at the central part of the cornea and deeply at the peripheral part. Power (diopter) is controlled by changing the total number of irradiation pulses without changing a ratio of the number of irradiation pulses at each position of the laser beam deviated from the optical axis L by the movement of the mirror 3 (for more detail, see Japanese Patent Application Unexamined Publication No. Hei8-66420 corresponding to U.S. Patent No. 5,800,424).

### (Ablation for Astigmatic Correction)

In the case of the astigmatic correction, the opening diameter of the circular aperture 7 is fixed, and the mirror 3 is moved stepwise to move (scan) the laser beam in the Gaussian distribution direction. At this time, the moving (scanning) direction of the laser beam of which the beam cross section is in the rectangular shape is adjusted by the rotation of the image rotator 5 so as to correspond to a flattest meridian direction of the astigmatism. Then, as the laser beam is moved (scanned) stepwise and the number of irradiation pulses at each position is increased at a proper ratio as nearing the peripheral part of the cornea, ablation for hyperopic astigmatic correction may be performed shallowly at the central part and deeply at the peripheral part in the flattest meridian direction. On the contrary, if the number of irradiation pulses at each position at the time of moving (scanning) the laser beam stepwise is increased as nearing the central part of the cornea (at this time, the moving (scanning) direction of the laser beam is adjusted so as to correspond to a steepest meridian direction of the astigmatism), ablation for myopic astigmatic correction may be performed deeply at the central part and shallowly at the peripheral part in the steepest meridian direction (for more detail, see Japanese Patent Application Unexamined Publication No. Hei8-66420 corresponding to U.S. Patent No. 5,800,424).

Further, in the case of the myopic astigmatic correction, the ablation may also be performed by the following method. The opening diameter of the circular aperture 7 is fixed, and the opening direction of the slit aperture 9 is previously adjusted so that its opening width is changed in the steepest meridian direction. As for the laser beam irradiation, as in the case of the above-described myopic correction, the laser beam is moved (scanned) in the Gaussian distribution direction by the movement of the mirror 3, and every time the laser beam has been provided one scan, the movement (scanning) direction of the laser beam is changed by the rotation of the image rotator 5 to perform approximately uniform ablation of the cornea within the opening of the slit aperture 9. These are performed every time the opening width of the slit aperture 9 is changed. Thereby, the ablation for the myopic astigmatic correction may be performed deeply at the central part of the cornea and shallowly at the peripheral part in the steepest meridian direction.

Next, the ablation for presbyopic correction in combination of the ablation for myopic correction and the ablation for hyperopic correction will be described. Hereinafter, description will be separately given on the ablation for hyperopic presbyopic correction and that for myopic presbyopic correction.

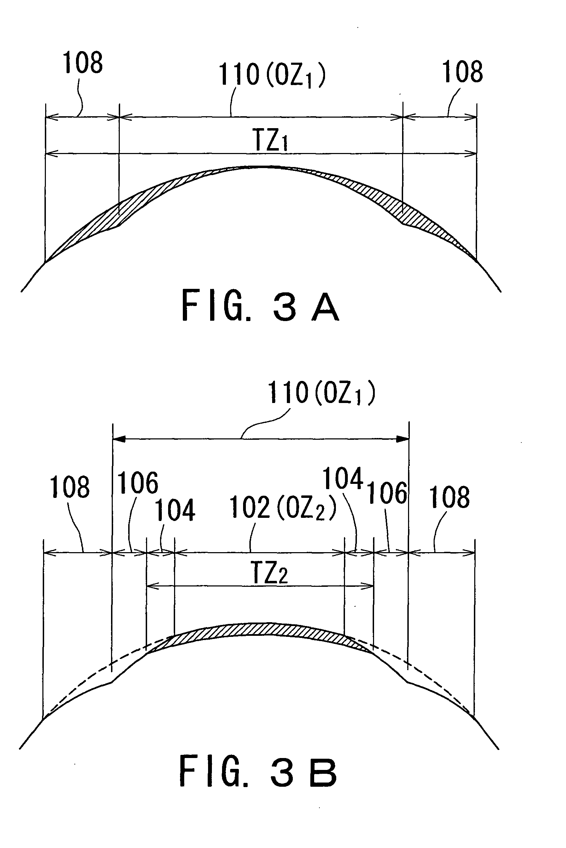

In the case of the hyperopic presbyopic correction, firstly, as a first stage, an area within an outer diameter of the near vision area 106 is set as an optical zone 110 and the ablation for hyperopic correction is performed thereon, as shown in Fig. 3A. In addition, the transition zone 108 outside the optical zone 110 is formed also utilizing the ablation for hyperopic correction. In Fig. 3A, a diagonally shaded portion is a portion to be ablated through the ablation for hyperopic correction. Incidentally, the transition zone 108 is formed by sequentially enlarging the opening diameter of the circular aperture 7 enlarged to a size of the optical zone 110, in accordance with the deviation of the laser beam with respect to the optical axis L.

Next, as a second stage, an area within the far vision area 102 in the central part of the area 110 after the ablation for hyperopic correction is set as an optical zone, and the ablation for myopic correction is performed thereon, as shown in Fig. 3B. Also, in a case where the blend zone 104 for the middle vision is provided between the far vision area 102 and the near vision area 106, the blend zone 104 is set as a transition zone and is formed by utilizing the ablation for myopic correction. In Fig. 3B, a diagonally shaded portion is a portion to be ablated through the ablation for myopic correction.

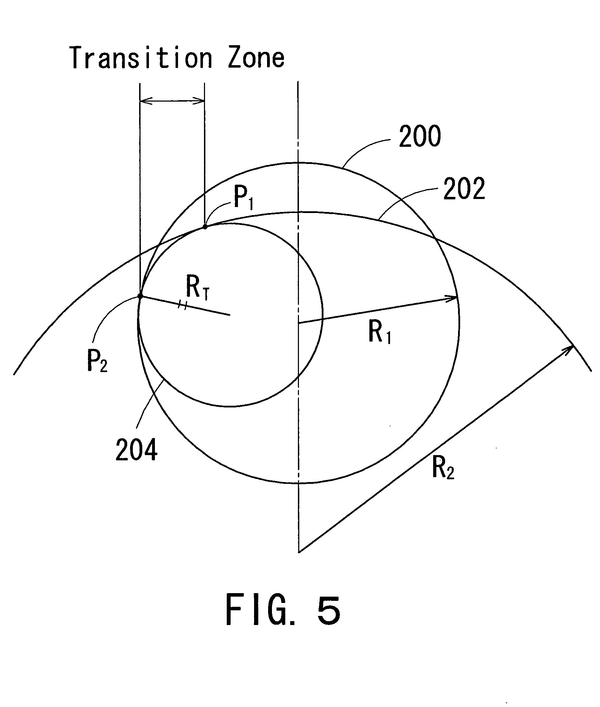

The transition zone formed by the ablation for myopic correction is in a curved shape inscribed in each curved surface of the cornea before and after the ablation. Owing to this, the non-ablation area and the ablation area are smoothly connected. Fig. 5 is a view illustrating a method for forming the transition zone by the ablation for myopic correction. An arc 200 indicates a curved surface of the cornea before the ablation having a radius of curvature R1, and an arc 202 indicates a curved surface of the cornea after the ablation having intended radius of curvature R2. Further, a circle 204 with a radius of curvature RT which inscribes in the arcs 200 and 202 at a point P1 on the arc 202 and a point P2 on the arc 200, respectively, determined by a forming position of the transition zone makes the curved shape of the transition zone. For detail of the method for forming the transition zone, please refer to Japanese Patent Application Unexamined Publication No. Hei6-189999 corresponding to U.S. Patent No. 5,445,633.

Besides, it is preferable that a size of the ablation for hyperopic correction is OZ₁ (a diameter of the optical zone 110) = about 6.5 mm, and TZ₁ (an outer diameter of the transition zone 108) = about 10.0 mm. In LASIK surgery where the ablation is performed after a corneal flap is formed, there is a case where TZ₁ is set small in accordance with a size of the corneal flap. On the other hand, it is preferable that a size of the ablation for myopic correction is OZ₂ (a diameter of the far vision area 102) = about 3.0 to 4.0 mm, and TZ₂ (an outer diameter of the blend zone 104) = about 4.0 to 5.0 mm. In addition, each of the ablation sizes for hyperopic correction and myopic correction are preferably determined based on a pupil size of the patient, respectively.

A correction amount for each ablation is set as follows. When desired refractive power after the operation is emmetropia, for the ablation for hyperopic correction at the first stage, the correction amount is set by near vision refractive power or far vision refractive power, and additional power. For the ablation for myopic correction at the second stage, the correction amount is set which corresponds to the additional power or a difference between the far vision refractive power and the near vision refractive power. When the desired refractive power after the operation is deviated from the emmetropia, each correction amount is adjusted by the deviation amount.

For example, when the emmetropia is desired with respect to the patient's eye of which the pre-operative far vision refractive power S (spherical power) = +1.0D, and the additional power = +2.25D, S = +3.25D is set for the correction amount for the ablation for hyperopic correction, and S = -2.25D is set for the correction amount for the ablation for myopic correction.

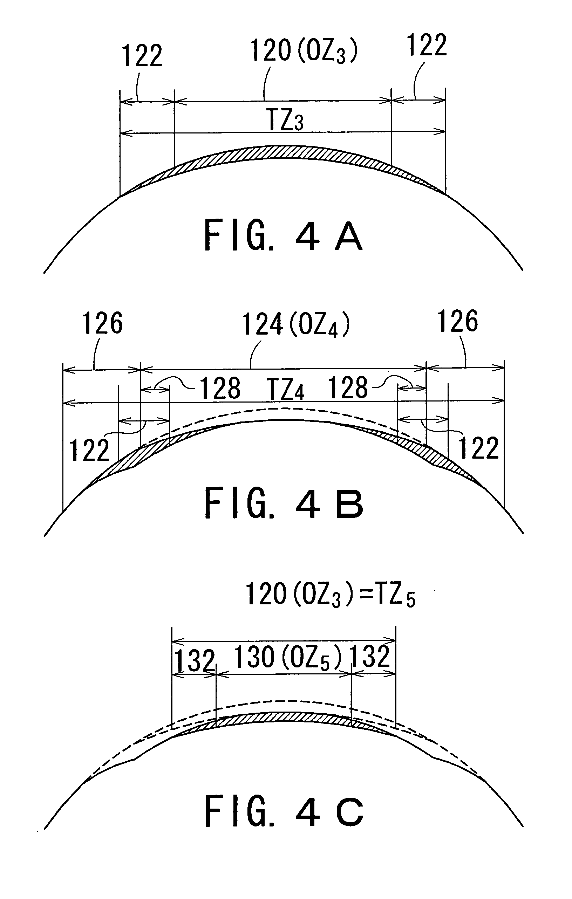

In the case of the myopic presbyopic correction, as a first stage, an optical zone 120 in the central part of the cornea and a transition zone 122 outside thereof are set, and the ablation for myopic correction is performed thereon, as shown in Fig. 4A. It is preferable that a size of the ablation for myopic correction is OZ₃ (a diameter of the optical zone 120) = about 5.0 to 6.0 mm, and TZ₃ (an outer diameter of the transition zone 122) = about 7.0 to 8.0 mm. In Fig. 4A, a diagonally shaded portion is a portion to be ablated through the ablation for myopic correction.

Next, as a second stage, an optical zone 124 and a transition zone 126 outside thereof are set, and the ablation for hyperopic correction is performed thereon, as shown in Fig. 4B. It is preferable that a size of the ablation for hyperopic correction is OZ₄ (a diameter of the optical zone 124) = about 6.5 mm, and TZ₄ (an outer diameter of the transition zone 126) = about 10.0 mm. Here, OZ₄ is made smaller than TZ₃. The transition zone 126 becomes an area corresponding to the transition zone 108 in Figs. 2A and 2B, and an area 128 where the transition zone 122 and the optical zone 124 overlap each other becomes an area corresponding to the near vision area 106 in Figs. 2A and 2B. In this area, the refractive power is gradually changed because of the formation of the transition zone 122. In Fig. 4B, a diagonally shaded portion is a portion to be ablated through the ablation for hyperopic correction.

Further, as occasion arises, as a third stage, additional ablation for myopic correction is performed on the area 120 after the ablation for hyperopic correction at the second stage, as shown in Fig. 4C. An optical zone 130 and a transition zone 132 outside thereof are set within the area 120, and the ablation for myopic correction is again performed thereon. It is preferable that a size of the ablation for myopic correction is OZ₅ (a diameter of the optical zone 130) = about 3.0 to 4.0 mm, and TZ₅ (an outer diameter of the transition zone 132) = about 5.0 to 6.0 mm. In this case, the optical zone 130 becomes an area corresponding to the far vision area 102 in Figs. 2A and 2B, and the transition zone 132 becomes an area corresponding to the blend zone 104 in Figs . 2A and 2B. In Fig. 4C, a diagonally shaded portion is a portion to be ablated through the ablation for myopic correction.

If the third stage is not performed, the optical zone 120 becomes an area corresponding to the far vision area 102 in Figs. 2A and 2B.

Besides, the far vision area in the central part of the cornea may be secured by performing the ablation for hyperopic correction (ablation in an annular shape) on an area except the optical zone 130 as shown in Fig. 4C at the second stage.

For the ablation for myopic correction at the first stage, the correction amount is set based on the far vision refractive power. For the ablation for hyperopic correction at the second stage, the correction amount corresponding to the additional power is set. If the ablation for hyperopic correction at the second stage causes somewhat myopia, the correction amount is set at the third stage so as to correct the deviation amount. For example, S = -0.75D may be set. If the third stage is not set, there is a case where the correction amount at the first stage or the second stage is adjusted.

At the time of each of the presbyopic corrections, the data on the pre-operative corneal shape, the data on the correction amount for each stage, and the data on the ablation size for each stage (OZ, TZ) are inputted by the data input unit 21. The calculation unit 22 calculates the post-ablative corneal shape for each stage based on the inputted data to obtain the data on the corneal ablation for each stage. Based on the data on the corneal ablation for each stage, in the laser irradiation for performing the ablation for myopic correction, data for controlling the opening diameter of the circular aperture 7 is determined. In the laser irradiation for performing the ablation for hyperopic correction, data for controlling the movement of the mirror 3 and the number of irradiation pulses are determined. The control unit 20 controls the laser irradiation system based on the determined control data. The laser irradiation for each stage is sequentially performed.

Besides, the laser irradiation system may be the one which uses a scanning mirror (which may be constituted by two galvano-mirrors) for scanning the laser beam formed into a small spot of about 0.1 to 1.0 mm two-dimensionally in the X and Y directions. In this case, the laser irradiation for the post-operative corneal shape shown in Fig. 2B may be implemented at once without combining each of the stages.

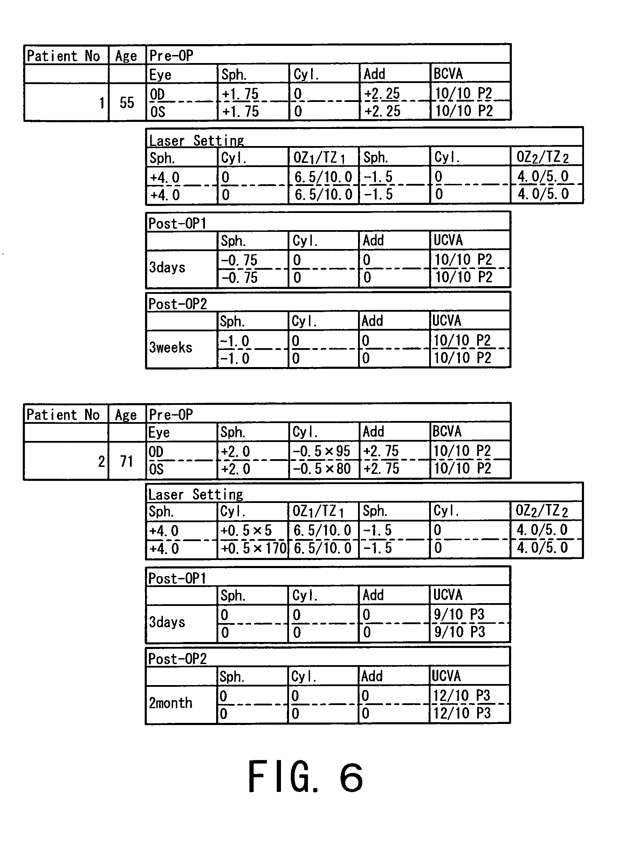

Fig. 6 shows results of the correction of a hyperopic presbyopic eye performed by the present apparatus. As to a patient No.1, the pre-operative far vision refractive powers of both eyes (OD, OS) were S = +1.75D, and the additional power = +2.25D. Thus, the ablation for hyperopic correction at the first stage was set as S = +4.0D, OZ₁ = 6.5 mm, TZ₁ = 10.0 mm. Further, the ablation for myopic correction at the second stage was set as S = -1.5D, OZ₂ = 4.0 mm, TZ₂ = 5.0 mm.

The post-operative far vision refractive power of the patient No.1 was somewhat weak as S = -0.75D three days after the operation, and S=-1.0D three weeks after the operation. However, for far vision eyesight (visual acuity) of a naked eye, 1.0 was obtained. Also for near vision eyesight (visual acuity) of the naked eye, vision capable of reading a character at P2 (which indicates visual acuity of the near vision, and the character becomes larger in the order of P2, P3, P4, P5) was obtained. Both of the obtained results were equivalent to those of corrected eyesight (visual acuity) by means of the spectacles before the operation.

As to a patient No.2, the pre-operative far vision refractive power of a right eye (OD) was S = +2.0D, C (cylindrical power) = -0.5D, A (a cylindrical axial angle) = 95°, and the additional power = +2.75D. The pre-operative far vision refractive power of a left eye (OS) was S = +2.0D, C=-0.5D, A = 80°, and the additional power = +2.75D. For the hyperopic presbyopic eye, as the cylindrical power is corrected through the ablation for hyperopic astigmatic correction, the cylindrical power is read by plus. In this case, the pre-operative far vision refractive power of the right eye is S = +1.5D, C = +0.5D, A = 5° , and the pre-operative far vision refractive power of the left eye is S = +1.5 D, C = +0.5D, A = 170° .

For the patient No. 2, the ablation for hyperopic correction at the first stage was set as S = +4.0D, OZ₁ = 6.5 mm, TZ₁ = 10.0 mm, and the correction for near vision was set slightly weak (by+0.25D) . Further, the ablation for myopic correction at the second stage was set as S = -1.5D, OZ₂ = 4.0 mm, TZ₂ = 5.0 mm, and the correction for far vision was set slightly strong (by -0.25D).

The post-operative far vision refractive power of the patient No.2 was 0D (emmetropia). The far vision eyesight of a naked eye three days after the operation was 0.9; however, 1.2 was obtained two months after the operation. Also, for near vision eyesight of the naked eye, vision capable of reading small characters up to P3 was obtained.

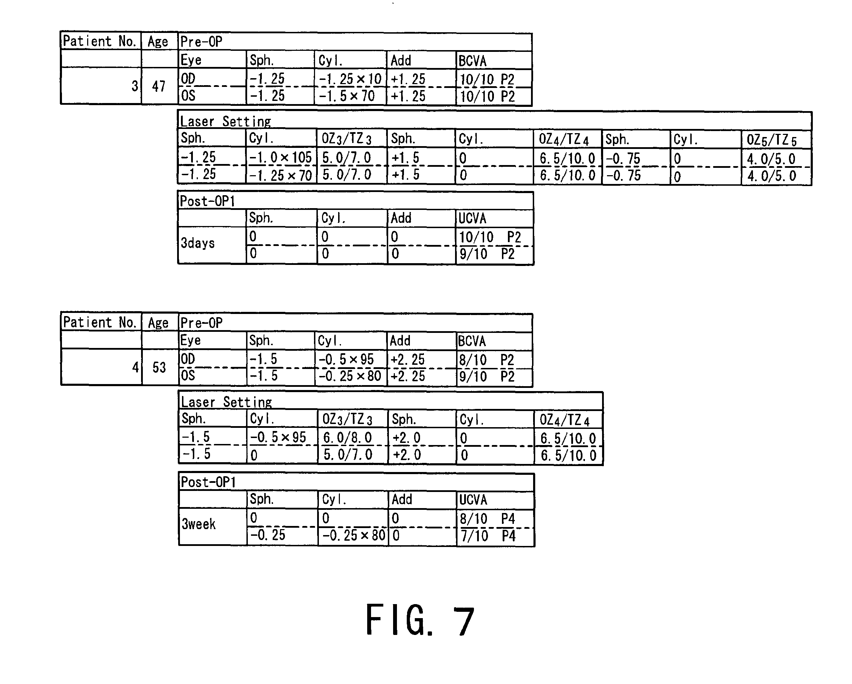

Fig. 7 shows results of the correction of the myopic presbyopic eye performed by the present apparatus. As to a patient No.3, the ablation for myopic correction at the first stage was set as S = -1.25D, OZ₃ = 5.0 mm, TZ₃ = 7.0 mm, that are the same as the pre-operative far vision refractive power. Further, the ablation for hyperopic correction at the second stage was set as S = +1.5D, OZ₄ = 6.5 mm, TZ₄ = 10.0 mm, that are slightly stronger than the pre-operative additional power. Furthermore, the ablation for myopic correction at the third stage was set as S = -0.75D, OZ₅ = 4.0 mm, TZ₅ = 5.0 mm. For the far vision eyesight of the naked eye and the near vision eyesight of the naked eye three days after the operation, favorable results were obtained.

As to a patient No.4, the ablation was set up to the second stage. In the ablation for myopic correction at the first stage, OZ₃ and TZ₃ of the right eye (OD) were made 1 mm larger than those of the patient No.3, respectively. Further, the ablation for hyperopic correction at the second stage was set as S = +2.0D, that was slightly weaker than the additive diopter. Other conditions were set in the same manner as those for the patient No.3. For the far vision eyesight of the naked eye, such results were obtained that were close to the vision obtained through the correction by means of the spectacles. Also for the near vision eyesight of the naked eye, favorable results were obtained.

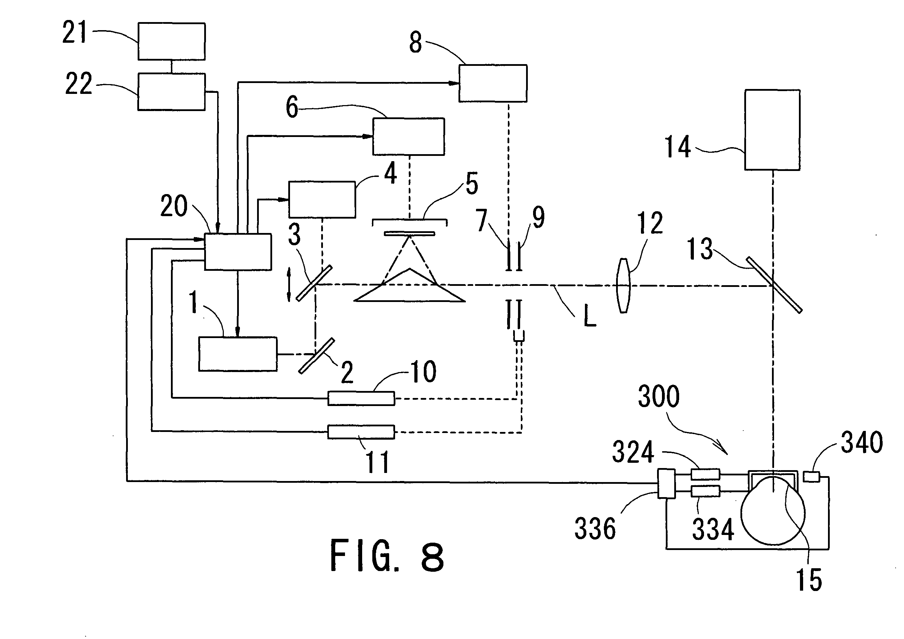

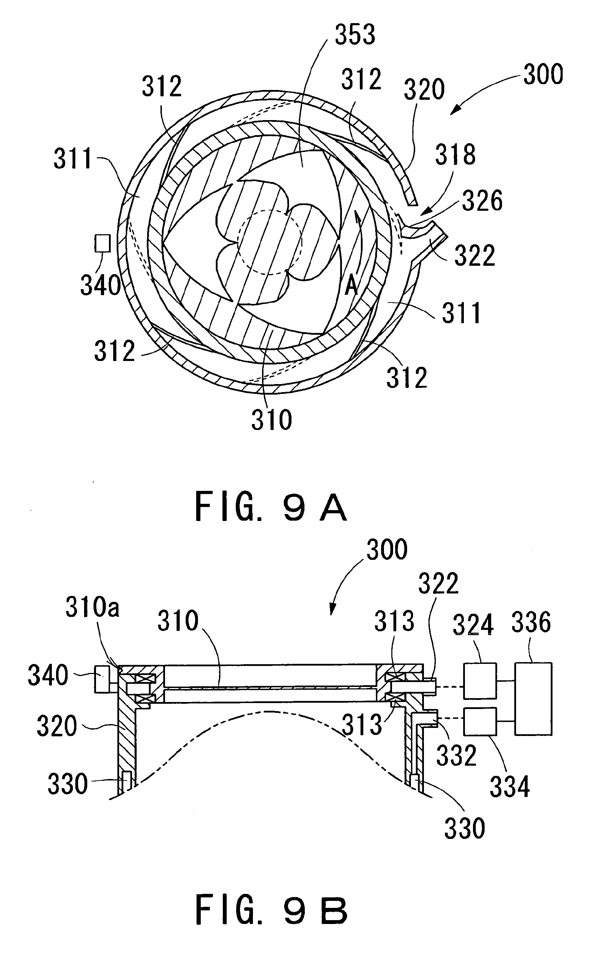

Next, a description will be given on another embodiment in which a mask plate having a light shielding area and an opening is disposed on the cornea 15, the far vision area 102 is formed (set) in the central part of the cornea, and the annular near vision area 106 is set outside thereof. Fig. 8 is a view showing a configuration in a case where the mask plate is disposed on the cornea. Reference numeral 300 is a mask unit having the mask plate. Figs. 9A and 9B are views showing a schematic configuration of the mask unit 300.

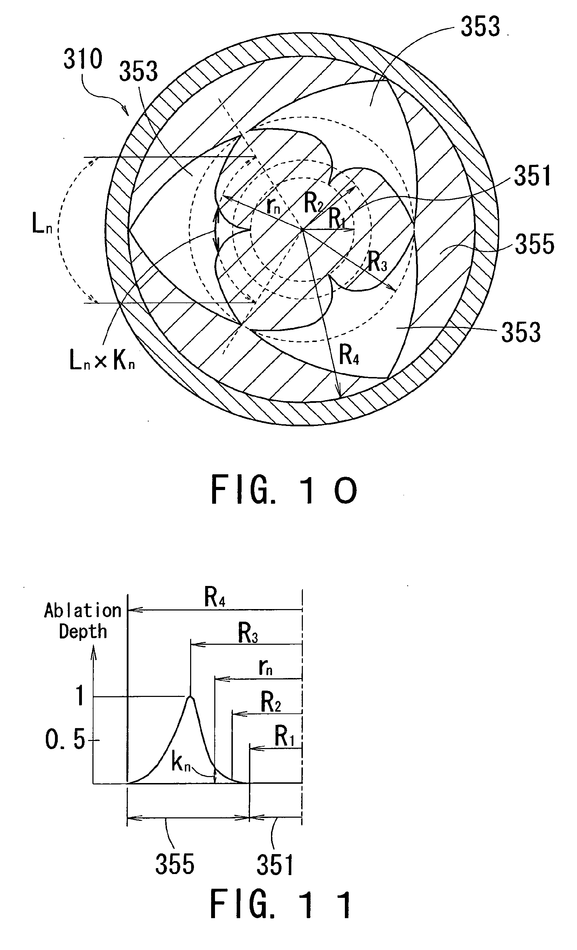

In a circular mask plate 310, an area for cutting off the laser beam and an opening for passing the laser beam are formed (see Fig. 10 for detail) . The mask plate 310 is rotatably held on a ring block 320 by two ring bearings 313 arranged above and below. The ring block 320 is used for placing the mask plate 310 on the cornea 15. Between a side face of an outer rim of the mask plate 310 and the ring block 320, a space 311 is formed. Further, four fins 312 are attached to the side face of the outer rim of the mask plate 310, at regular intervals. The fins 312 are made of an elastic material such as rubber, and the tip portions thereof are in contact with the inner wall of the ring block 320.

Provided to the ring block 320 is an aspiration vent 322 which is connected to an aspiration source 324 via a tube. A convex part 326 for narrowing an aspiration path and enabling the fins 312 to pass is formed on the inner wall of the ring block 320 which is positioned near the aspiration vent 322. Further, an inflow vent 318 for taking in air into the space 311 is provided near the convex part 326. Besides, the space 311 is almost sealed except for the inflow vent 318 and the aspiration vent 322. If the aspiration source 324 inhales the air in the space 311, a negative pressure is developed in the space 311. Due to such aspiration pressure, the fins 312 near the aspiration vent 322 are pulled, and the mask plate 310 rotates in the direction of the arrow A shown in Fig. 9A. When the fins 312 collide against the convex part 326, they are bended to pass the convex part 326.

Besides, a rotation mechanism of the mask plate 310 is not limited to the above-mentioned constitution. For example, it may have a constitution where a plurality of blades are provided to the side of the outer rim of the mask plate 310, and compressed air is sent to the blades to rotate the mask plate 310.

The ring block 320 is fixed while its lower end portion is in contact with a screla of the patient' s eye. On the lower end portion brought into contact with the screla, a groove 330 for adsorption is formed, and the groove 330 is connected to an aspiration vent 332. The aspiration vent 332 is connected to an aspiration source 334 via a tube. The aspiration source 334 provides an aspiration pressure, so that the ring block 320 is fixed to the patient's eye.

It is preferable that a sensor 340 for detecting the number of rotation of the mask plate 310 is provided to the mask unit 300. The sensor 340 is comprised of a light source for emitting detection light and a photodetector, and photo-receives the detection light reflected by a groove 310a provided on the mask plate 310 to detect the number of rotation of the mask plate 310. A control unit 336 in the mask unit 300 controls driving of the aspiration sources 324 and 334.

In the presbyopic correction using the mask unit 300, when the laser beam is irradiated while rotating the mask plate 310, the ablation is not performed on the central part of the cornea in order to secure the far vision area 102, and the ablation for securing the annular near vision area 106 and the transition zone 108 is performed outside the central part of the cornea as shown in Figs. 2A and 2B. Further, preferably, the ablation for forming the blend zone 104 for middle vision is performed between the far vision area 102 and the near vision area 106.

As for the size of each area, for example, the far vision area 102 is 3 mm in diameter (a radius R1 = 1.5 mm) , the blend zone 104 is 4 mm in outer diameter (a radius R2 = 2 mm) , the near vision area is 6. 5 mm in outer diameter (a radius R3 = 3. 25 mm) , and the transition zone 108 is 10 mm in outer diameter (a radius R4 = 5 mm). This is only an example, and the size suitable for the patient may be used.

Fig. 10 is a view illustrating a shape of the mask plate 310. The mask plate 310 has a light shielding area 351 having the radius R1 corresponding to the far vision area 102 in the central part, and an area 355 outside the light shielding area in which three openings 353 in the same shape are formed at regular intervals. The area 355 is an area for securing the blend zone 104 (radius R1 to R2), the near vision area 106 (radius R2 to R3), and the transition zone 108 (radius R3 to R4). The near vision area 106 is an area on which the ablation for a hyperopic shift in the amount of the additional power necessary for the presbyopic correction with respect to the far vision area 102 is performed. The blend zone 104 is an area on which the ablation is performed so as to be progressively changed to the additional power.

As for the openings 353 formed in the area 355, in the range of the blend zone 104 (radius R1 to R2) and the near vision area 106 (radius R2 to R3), a ratio of a circumferential length of the opening 353 at a radius position of the mask plate 310 is made smaller as nearing a center of the mask plate 310. An example for determining it will be described hereinafter. The ablation by the amount of the additional power as the amount for the near vision correction (for example, 1D) is assumed to be performed on the near vision area 106, and data on ablation depth distribution of the near vision area 106 of radius R2 to R3 and that of the blend zone 104 of radius R1 to R2 are previously obtained (see Fig. 11). Maximum ablation depth for the radius R3 being an outermost circumference of the near vision area 106 is set as 1, and a ratio Kn of the ablation depth at a certain radius position rn with respect to the maximum ablation depth is obtained. The circumferential length of the opening 353 with respect to the circumferential length Ln at the radius position rn is obtained by an expression Ln × Kn. This is obtained for the range R1 to R3, so that the shape of the opening 353 in the range is determined.

On the contrary, in the range of the transition zone 108 (radius R3 to R4) of the opening 353, the ratio of the circumferential length of the opening 353 at the radius position of the mask plate 310 is made smaller as nearing an outer rim of the mask plate 310. As for the transition zone 108, in a similar manner, data on ablation depth distribution in radius R3 to R4 is previously obtained, and the circumferential length of the opening 353 at the certain radius position is obtained based on the ratio Kn of the ablation depth at the certain radius position to determine the shape of the opening 353. Incidentally, since the mask plate 310 shown in Fig. 10 is provided with three openings 353, the circumferential length of the opening 353 is preferably determined while considering the size of connecting portions.

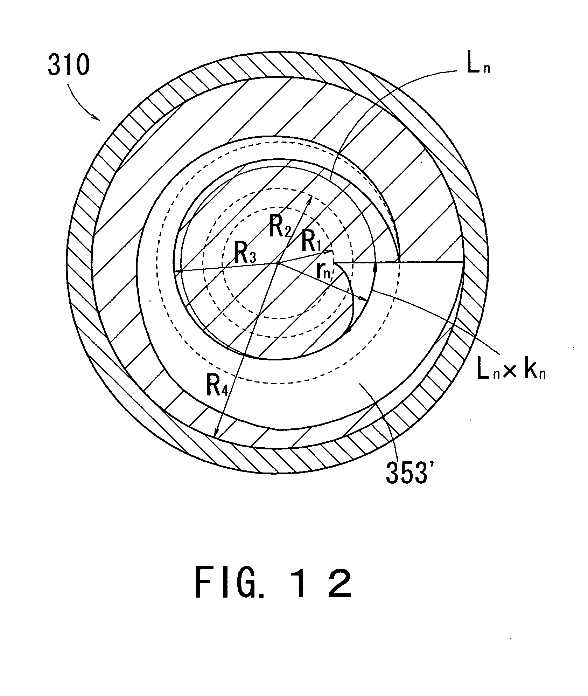

Fig. 12 is an example of the mask plate 310 in which one opening is formed. As to a shape of the opening 353', as mentioned above, its circumferential length at the radius position rn is obtained as Ln x Kn. In this example, in order to make the light shielding area divided in two to be rotatable, the light shielding area is formed on a transparent member which transmits a laser beam. (The opening 353' is a part on which the light shielding area is not formed.)

Presbyopic corrective surgery using the mask unit 300 will be described hereinafter. In a case where the correction is performed on the near vision area 106 by the amount of the additional power for near vision without correcting the far vision area 102, the patient's eye is observed with the observation optical system 14, and the mask unit 300 is placed above it. After the ring block 320 is adsorbed and fixed to the patient' s eye by driving the aspiration source 334, the mask plate 310 is rotated by driving the aspiration source 334. At this time, the sensor 340 monitors the number of rotation of the mask plate 310, and the control unit 336 controls the aspiration pressure of the aspiration source 324 so that a rotation period of the mask plate 310 is synchronized with the number of pulses of the laser beam emitted from the laser source 1 (for example, 60Hz).

An irradiation method which enables the ablation with substantially uniform depth by the scan of the previously-described laser beam of which the beam cross section is the rectangular shape is used for the laser irradiation. Hereinafter, the description will be given on the irradiation method. The laser beam is moved (scanned) in the Gaussian distribution direction by the movement of the mirror 3 and is superimposed. When the laser beam is irradiated while rotating the mask plate 310, the cornea is ablated by the laser beam passed through the opening 353. Every time one scan has been provided, the control unit 20 adjusts and differentiates timing of emission of the pulsed laser beam from the laser source 1. Thereby, a rotation position of the opening 353 on the cornea is deviated, and the irradiation position of the laser beam having passed through the opening 353 is also deviated. The ablation with the laser beam is superimposed, and the ablation depth at each of the radius positions is changed while being related to the number of scans and the circumferential length of the opening 353 at the radius position. The additional power for near vision may be controlled by changing the number of scans (laser irradiation time). The data input unit 21 inputs the additional power for near vision, and the control unit 20 (or the calculation unit 22) obtains the control data on the laser irradiation system. As a consequence, the far vision area 102 on which the ablation is not performed is secured in the central part of the cornea, and the annular near vision area 106, the transition zone 108 and the blend zone 104 are formed outside the far vision area 102.

Besides, the control of the correction amount may be performed by preparing several openings 353 with different shapes according to the additional power for near vision, instead of changing the number of scans (laser irradiation time).

In addition, in a case where a laser irradiation system capable of irradiating the laser beam broader than the mask plate 310 is used, instead of the laser irradiation system which irradiates the laser beam of which the beam cross section is in the rectangular shape as mentioned above, the number of pulses of the laser beam is adjusted with respect to the number of rotation of the mask plate 310, so that the rotation position of the opening 353 at the time of the laser irradiation may be deviated.

In the case of the hyperopic presbyopic correction, the ablation for hyperopic correction as mentioned above is performed before or after the ablation using the mask unit 300. At the time of the surgery, the data on the pre-operative corneal shape, the data on the correction amount for far vision, the data on the additional power for near vision, and data on a size of the optical zone for the ablation for hyperopic correction are inputted. In this case, it is preferable that the size of the optical zone is set to include the near vision area 106 for the presbyopic correction by the mask plate 310, and the ablation for the transition zone is set around it. The control data for the laser irradiation system is prepared based on the inputted data. Incidentally, the control data may be calculated by obtaining the corneal shape at each of the steps of the ablation for hyperopic correction and the ablation performed by the mask unit 300.

Further, also in the case of the myopic presbyopic correction, the ablation for myopic correction as mentioned above is performed before or after the ablation using the mask unit 300. It is also preferable that the size of the optical zone for the ablation for myopic correction is set to include the near vision area 106, and the ablation for the transition zone is set around it.

As described above, the presbyopic correction for making the post-operative condition of visibility favorable may be easily allowed.

The foregoing description of the preferred embodiments of the invention has been presented for purposes of illustration and description. It is not intended to be exhaustive or to limit the invention to the precise form disclosed, and modifications and variations are possible in the light of the above teachings or may be acquired from practice of the invention. The embodiments chosen and described in order to explain the principles of the invention and its practical application to enable one skilled in the art to utilize the invention in various embodiments and with various modifications as are suited to the particular use contemplated. It is intended that the scope of the invention be defined by the claims appended hereto.

## Claims

1. An apparatus for calculating data on corneal ablation for corneal surgery for correcting a refractive error by ablating corneal tissue with a laser beam, the apparatus comprising:
first input means for inputting a pre-operative corneal shape;
second input means for inputting a correction amount for far vision and additional power for near vision;
setting means for setting a far vision area in a central part of a cornea and an annular near vision area outside the far vision area; and
calculation means for obtaining the data on the corneal ablation based on the inputted pre-operative corneal shape, the inputted correction amount for far vision and the inputted additional power for near vision, and the set far vision area and the set near vision.

2. The apparatus for calculating data on corneal ablation according to claim 1, wherein the setting means further sets an area for smoothly connecting the far vision area and the near vision area.

3. The apparatus for calculating data on corneal ablation according to claim 1, wherein the calculation means obtains data on ablation for myopic correction ablating a central part of the cornea deeply and a peripheral part of the cornea shallowly, and data on ablation for hyperopic correction ablating the central part of the cornea shallowly and the peripheral part of the cornea deeply, respectively.

4. A corneal surgery apparatus for correcting a refractive error by ablating corneal tissue with a laser beam, the apparatus comprising:
a laser irradiation system having an irradiation optical system for irradiating the laser beam onto a cornea;
first input means for inputting a pre-operative corneal shape;
second input means for inputting a correction amount for far vision and additional power for near vision;
setting means for setting a far vision area in a central part of the cornea and an annular near vision area outside the far vision area;
calculation means for obtaining data on corneal ablation based on the inputted pre-operative corneal shape, the inputted correction amount for far vision and the inputted additional power for near vision, and the set far vision area and the set near vision area; and
control means for controlling the laser irradiation system based on the obtained data on the corneal ablation.

5. The corneal surgery apparatus according to claim 4, wherein the setting means further sets an area for smoothly connecting the far vision area and the near vision area.

6. The corneal surgery apparatus according to claim 4, wherein
the laser irradiation system includes
ablation means for myopic correction, for ablating the central part of the cornea deeply and a peripheral part of the cornea shallowly, and
ablation means for hyperopic correction, for ablating a central part of the cornea shallowly and the peripheral part of the cornea deeply;
the calculation means obtains data on ablation for myopic correction and data on ablation for hyperopic correction, respectively; and
the control means controls each ablation means based on each data on the ablation and performs presbyopic correction by combining each ablation.

7. The corneal surgery apparatus according to claim 4, wherein
the laser irradiation system includes
beam limiting means for variably limiting an area to be ablated with the laser beam,
beam rotating means for rotating the laser beam about a central optical axis of the irradiation optical system, and
beam deviating means for deviating the laser beam with respect to the central optical axis; and
the control means performs presbyopic correction by controlling the beam limiting means, the beam rotating means and the beam deviating means.

8. The corneal surgery apparatus according to claim 4, wherein the setting means includes a mask plate having a light shielding area corresponding to the far vision area in a central part, and a first opening corresponding to the near vision area outside the light shielding area, the first opening having a shape where a ratio of a circumferential length at a radius position of the mask plate is made smaller as nearing a center of the mask plate.

9. The corneal surgery apparatus according to claim 8, wherein the mask plate has a second opening outside the first opening, the second opening having a shape where the ratio of the circumferential length at the radius position of the mask plate is made smaller as nearing an outer rim of the mask plate, and being connected to the first opening.

10. The corneal surgery apparatus according to claim 8, further comprising mask rotating means for rotating the mask plate on the cornea.

11. A corneal surgery apparatus for correcting a refractive error by ablating corneal tissue with a laser beam, the apparatus comprising:
a laser irradiation system having
a laser source, and
an irradiation optical system for irradiating
the laser beam onto a cornea; and
a mask plate having a light shielding area corresponding to a far vision area in a central part, and a first opening corresponding to an annular near vision area outside the light shielding area, the first opening having a shape where a ratio of a circumferential length at a radius position of the mask plate is made smaller as nearing a center of the mask plate.

12. The corneal surgery apparatus according to claim 11, wherein the mask plate has a second opening outside the first opening, the second opening having a shape where the ratio of the circumferential length at the radius position of the mask plate is made smaller as nearing an outer rim of the mask plate, and being connected to the first opening.

13. The corneal surgery apparatus according to claim 11, wherein the first opening in the mask plate has a shape where the circumferential length at the radius position of the mask plate is determined based on a ratio of ablation depth when desired additional power for near vision is secured.

14. The corneal surgery apparatus according to claim 11, further comprising mask rotating means for rotating the mask plate on the cornea.

15. The corneal surgery apparatus according to claim 14, further comprising control means for controlling at least one of the laser irradiation system and mask rotating means to adjust the number of pulses of the laser beam and the number of rotation of the mask plate.
